# EUROPÄISCHE PATENTSCHRIFT

(11) **EP 1 609 362 B1**
(45) Veröffentlichungstag und Bekanntmachung des Hinweises auf die Patenterteilung: **18.03.2015**
(21) Anmeldenummer: 05021260.4
(22) Anmeldetag: 08.05.1995
(51) Int. Cl.: A01N 61/00, A01N 51/00

(54) **Nicht-systemische Bekämpfung von Parasiten**
Non-systemic control of parasites
Lutte non-systémique contre les parasites

(30) Priorität: 20.05.1994 DE 4417742
(43) Veröffentlichungstag der Anmeldung: 28.12.2005
(62) Teilanmeldung aus: 95106925.1
(73) Patentinhaber: Bayer Intellectual Property GmbH, 40789 Monheim (DE)
(72) Erfinder: Dorn, Hubert, Dr., 42115 Wuppertal (DE); Hopkins, Terence, Dr., Tamborine Qld. (AU)
(74) Vertreter: von Renesse, Dorothea

(56) Entgegenhaltungen:
- EP-A1- 0 483 052
- EP-A2- 0 268 915
- EP-A2- 0 413 610
- WO-A1-93/24002
- CH-A- 114 863
- DE-C- 587 853
- US-A- 4 742 060
- US-A- 4 874 753
- DATABASE WPI Week 199600 Thomson Scientific, London, GB; AN 1966-10652F XP002555799 & JP 63 000244 B (TANABE SEIYAKU) 6. Januar 1988 (1988-01-06)
- A.ELBERT ET AL.: "Imidacloprid-a new systemic insecticide" PFLANZENSCHUTZ-NACHRICHTEN BAYER, Bd. 44, Nr. 2, 1991, Seiten 113-136, XP000566033 LEVERKUSEN,DE; ISSN: 0340-1723
- A.ELBERT ET AL.: "Imidacloprid, a Novel Nitromethylene Analogue Insecticide for Crop Protection" BRIGHTON CROP PROTECTION CONFERENCE-PESTS AND DISEASES, Nr. 1, 1990, Seiten 21-28, XP000566032 THORNTON HEATH, GB; ISSN: 0955-1506
- DATABASE CA [Online] CHEMICAL ABSTRACTS SERVICE, COLUMBUS, OHIO, US; 1984, JONES, S. W. ET AL: "Interaction of insecticides with acetylcholine receptors" XP002555797 gefunden im STN Database accession no. 91:1323 & NEUROTOXICOL. INSECTIC. PHEROMONES, [PROC. SYMP.] , MEETING DATE 1978, 259-75. EDITOR(S): NARAHASHI, TOSHIO. PUBLISHER: PLENUM, NEW YORK, N. Y. CODEN: 40IRAB, 1979,
- DATABASE CA [Online] CHEMICAL ABSTRACTS SERVICE, COLUMBUS, OHIO, US; 1984, O'BRIEN, R. D.: "The interaction of pesticides with gates, receptors and enzymes of the nervous system" XP002555798 gefunden im STN Database accession no. 91:69959 & ADV. PESTIC. SCI., PLENARY LECT. SYMP. PAP. INT. CONGR. PESTIC. CHEM., 4TH , MEETING DATE 1978, VOLUME 3, 449-57. EDITOR(S): GEISSBUEHLER, HANS. PUBLISHER: PERGAMON, OXFORD, ENGL. CODEN: 40TYAJ, 1979,

## Beschreibung

Die vorliegende Erfindung betrifft die nicht-systemische Bekämpfung von parasitierenden Insekten aus der Gruppe der Flöhe, Läuse und Fliegen an Menschen und Tieren mittels Agonisten oder Antagonisten der nicotinergen Acetylcholinrezeptoren von Insekten, nämlich Imidacloprid.

Agonisten oder Antagonisten der nicotinergen Acetylcholinrezeptoren von Insekten sind bekannt. Zu ihnen gehören die Nicotinyl-Insektizide und ganz besonders die Chlornicotinyl-Insektizide. Es ist auch bekannt, daß diese Verbindungen hervorragend gegen pflanzenschädigende Insekten wirken. Die systemische Wirkung dieser Verbindungen in Pflanzen gegen pflanzenschädigende Insekten ist ebenfalls bekannt.

Aus der PCT-Anmeldung WO 93/24 002 ist bekannt, daß sich bestimmte 1-[N-(Halo-3-pyridylmethyl)]-N-methylamino-1-alkylamino-2-nitroethylen-Derivate zur systemischen Anwendung gegen Flöhe bei Haustieren eignen. Bei dieser Art der Anwendung wird der Wirkstoff dem Haustier oral oder parenteral z.B. durch Injektion verabreicht und gelangt so in die Blutbahn des Haustieres. Die Flöhe nehmen den Wirkstoff dann beim Blutsaugen auf. Als ungeeignet zur Bekämpfung der Flöhe an Haustieren wird nach WO 93/24 002 die nicht-systemische Art der Anwendung dargestellt.

Es wurde nun überraschenderweise gefunden, daß sich gerade Imidacloprid in Mischung mit den synthetischen Pyrethroiden Flumethrin oder Permethrin besonders zur nicht-systemischen Bekämpfung parasitierender Insekten wie Flöhen, Läusen oder Fliegen an Menschen und Tieren eignet.

Agonisten oder Antanogisten der nicotinogen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17659, 91/4965; Französische Anmeldung Nr. 2 611 114; Brasilianische Anmeldung Nr. 8803621.

Auf die in diesen Publikationen beschriebenen Methoden, Verfahren, Formeln und Definitionen sowie auf die darin beschriebenen einzelnen Präparationen und Verbindungen wird hiermit ausdrücklich Bezug genommen.

Imidacloprid (1-[(6-Chlor-3-pyridinyl)methyl]-N-nitro-2-imidazolidinium) hat die folgende Formel

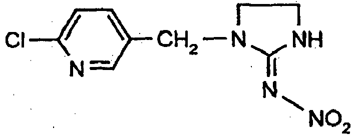

Der Wirkstoff eignet sich bei günstiger Warmblütertoxizität zur Bekämpfung von parasitierenden Insekten, die am Menschen und in der Tierhaltung und Tierzucht bei Haus- und Nutztieren sowie Zoo-, Labor-, Versuchs- und Hobbytieren vorkommen. Sie ist dabei gegen alle oder einzelne Entwicklungsstadien der Schädlinge sowie gegen resistente und normal sensible Arten der Schädlinge wirksam.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp.

Besonders hervorgehoben sei die Wirkung gegen Siphonaptera, insbesondere gegen Flöhe.

Bevorzugt genannt seien folgende Zieltiere:
Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.
Zu Labor- und Versuchstieren gehören Mäuse, Ratten, Meerschweinchen, Goldhamster, Hunde und Katzen.
Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen dermal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die dermale Anwendung geschieht z.B. in Form des Badens, Tauchens (Dippen), Sprühens (Sprayen), Aufgießens (pour-on oder spot-on), Waschens, Schamponierens, Begießens, Einpudems.

Geeignete Zubereitungen sind:
Lösungen oder Konzentrate zur Verabreichung nach Verdünnung, Lösungen zum Gebrauch auf der Haut, Aufgußformulierungen, Gele;
Emulsionen und Suspensionen zur dermalen Anwendung sowie halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, wirkstoffhaltige Formkörper.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht.

Die Lösungen werden hergestellt, indem der Wirkstoff in einem geeigneten Lösungsmittel gelöst wird und evtl. Zusätze wie Lösungsvermittler, Säuren, Basen, Puffersalze, Antioxidantien, Konservierungsmittel zugefügt werden.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester. Konservierungsmittel sind: Benzylalkohol, Trichlorbutanol, p-Hydroxybenzoesäureester, n-Butanol.

Es kann vorteilhaft sein, bei der Herstellung der Lösungen Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele, die auf die Haut aufgetragen oder aufgestrichen werden, werden hergestellt indem Lösungen, die wie oben beschrieben hergestellt worden sind, mit so viel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, Antioxidantien, Lichtschutzmittel, Haftmittel zugefügt.

Als Lösungsmittel seien genannt: Wasser, Alkanole, Glycole, Polyethylenglycole, Polyprppylenglycole, Glycerin, aromatische Alkohole wie Benzylalkohöl, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Hilfsstoffe sind auch spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphor-säureester-monoethanolaminsalz; kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen zur dermalen Verabreichung unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Anwendungsfertige Zubereitungen enthalten den Wirkstoff in Konzentrationen von 1 ppm - 20 Gewichtsprozent, bevorzugt von 0,01 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im Allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Besonders hervorgehoben sei die Anwendung über Formkörper. Formkörper sind u.a. Halsbänder, Anhänger für Halsbänder (Medaillons), Ohrmarken, Bänder zur Befestigung an Gliedmaßen oder Körperteilen, Klebestreifen und -folien, Abziehfolien.

Besonders hervorgehoben seien Halsbänder und Medaillons.

Für die Herstellung der Formkörper kommen thermoplastische und flexible hitzehärtbare Kunststoffe sowie Elastomere und thermoplastische Elastomere in Frage. Als solche seien genannt Polyvinylharze, Polyurethane, Polyacrylate, Epoxyharze, Cellulose, Cellulosederivate, Polyamide und Polyester, die mit den obengenannten Wirkstoff ausreichend verträglich sind. Die Polymeren müssen eine ausreichende Festigkeit und Biegsamkeit haben, um beim Formen nicht zu reißen oder brüchig zu werden. Sie müssen von ausreichender Haltbarkeit sein, um gegen normale Abnutzung beständig zu sein. Außerdem müssen die Polymere eine ausreichende Wanderung des Wirkstoffs an die Oberfläche des Formkörpers zulassen.

Zu den Polyvinylharzen gehören Polyvinylhalogenide, wie Polyvinylchlorid, Polyvinylchlorid-Vinylacetat und Polyvinylfluorid; Polyacrylat- and Polymethacrylatester, wie Polymethylacrylat und Polymethylmethacrylat; und Polyvinylbenzole, wie Polystyrol und Polyvinyltoluol. Besonders hervorgehoben sei Polyvinylchlorid.

Für die Herstellung der Formkörper auf der Basis Polyvinylharz sind die Weichmacher geeignet, die üblicherweise zum Weichmachen von festen Vinylharzen verwendet werden. Der zu verwendende Weichmacher hängt von dem Harz und seiner Verträglichkeit mit dem Weichmacher ab. Geeignete Weichmacher sind beispielsweise Ester von Phosphorsäure, wie Ester von Phthalsäure, wie Dimethylphthalat und Dioctylphthalat, und Ester von Adipinsäure, wie Diisobutyladipat. Es können auch andere Ester, wie die Ester von Azelainsäure, Maleinsäure, Ricinolsäure, Myristinsäure, Palmitinsäure, Ölsäure, Sebacinsäure, Stearinsäure und Trimellithsäure, sowie komplexe lineare Polyester, polymere Weichmacher und epoxydierte Sojabohnenöle verwendet werden. Die Menge des Weichmachers beträgt etwa 10 bis 50 Gew.%, vorzugsweise etwa 20 bis 45 Gew.-% der gesamten Zusammensetzung.

In den Formkörpern können noch weitere Bestandteile, wie Stabilisierungsmittel, Schmiermittel, Füllstoffe und Färbematerialien, enthalten sein, ohne daß dadurch die grundlegenden Eigenschaften der Zusammensetzung verändert werden. Geeignete Stabilisierungsmittel sind Antioxydationsmittel und Mittel, die die Bänder vor ultravioletter Strahlung und unerwünschtem Abbau während der Bearbeitung, wie Strangpressen schützen. Einige Stabilisierungsmittel, wie epoxydierte Sojabohnenöle, dienen außerdem als sekundäre Weichmacher. Als Schmiermittel können beispielsweise Stearate, Stearinsäure und Polyethylene mit niedrigem Molekulargewicht verwendet werden. Diese Bestandteile können in einer Konzentration bis zu etwa 5 Gew.-% der gesamten Zusammensetzung verwendet werden.

Bei der Herstellung der Formkörper auf Vinylbasis werden die verschiedenen Bestandteile nach bekannten Verfahren gemischt und nach bekannten Strangpreß- oder Spritzgußverfahren formgepreßt.

Die Wahl des Verarbeitungsverfahrens zur Herstellung der Formkörper richtet sich technisch grundsätzlich nach den rheologischen Eigenschaften des Bandmaterials und der Form des gewünschten Bandes. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenden rheologischen Zuständen unterteilen. Danach kommen für viskose Bandmaterialien Gießen, Pressen, Spritzgießen und Auftragen und für elastoviskose Polymere Spritzgießen, Strangpressen (Extrudieren), Kalandrieren, Walzen und gegebenenfalls Kanten in Frage. Nach Art der Formgebung eingeteilt, lassen sich die erfindungsgemäßen Formkörper durch Gießen, Tauchen, Pressen, Spritzgießen, Extrudieren, Kalandrieren, Prägen, Biegen, Tiefziehen etc. herstellen.

Diese Verarbeitungsverfahren sind bekannt und bedürfen keiner näheren Erklärung. Im Prinzip gelten für andere Polymere die Erläuterungen, die oben beispielhaft für Polyvinylharze gemacht wurden.

Die als Trägermaterial dienenden Polyurethane werden in an sich bekannter Weise durch Umsetzung von Polyisocyanaten mit höhermolekularen, mindestens zwei gegenüber Isocyanaten reaktionsfähigen Gruppen aufweisenden Verbindungen sowie gegebenenfalls niedermolekularen Kettenverlängerungsmitteln und/oder monofunktionellen Kettenabbrechern hergestellt.

Als Ausgangskomponenten bei der Herstellung der Polyurethane kommen aliphatische, cycloaliphatische, araliphatische, aromatische und heterocyclische Polyisocyanate in Betracht, wie sie z.B. von W. Siefken in Liebig's Annalen der Chemie, 562, Seiten 75 bis 136, beschrieben werden. Beispielhaft seien genannt: Ethylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Verbindungen, 1-Isocyanato-3,3,5.-trimethyl-5-isocyanatomethyl-cyclohexan (s. DE-AS 202 785 und US-PS 3 401 190), 2,4- und 2,6-Hexahydro-toluylen-diisocyanat sowie beliebige Gemische dieser Verbindungen; Hexahydro-1,3-und/oder -1,4-phenylen-diisocyanat, Perhydro-2,4'-und/oder -4,4'-diphenylmethan-diisocyanat, 1,3- und 1,4-Phenylendiisocyanat, 2,4- und 2,6-Toluylendiisocyanat sowie beliebige Gemische dieser Verbindungen; Diphenylmethan-2,4'-und/oder -4,4'-diisocyanat, Naphthylen-1,5-diisocyanat, Triphenylmethan-4,4', 4"-triisocyanat, Polyphenyl-polymethylen-polyisocyanate, wie sie durch Anilin-Formaldehyd-Kondensation und anschließende Phosgenierung erhalten und z.B. in den GB-PSen 874 430 und 848 671 beschrieben werden; m- und p-Isocyanatophenol-sulfonyl-isocyanate gemäß der US-PS 3 454 606; perchlorierte Arylpolyisocyanate, wie sie z.B. in der DE-AS 1 157 601 und in der US-PS 3 277 138 beschrieben werden; Carbodiimidgruppen aufweisende Polyisocyanate, wie sie in der DE-PS 1 092 007 und in der US-PS 3 152 162 beschrieben werden; Diisocyanate, wie sie in der US-PS 3 492 330 beschrieben werden; Allophanatgruppen aufweisende Polyisocyanate, wie sie z.B. in der GB-PS 994 890, der DE-PS 761 626 und der veröffentlichten holländischen Patentanmeldung 7 102 524 beschrieben werden; Isocyanuratgruppen aufweisende Polyisocyanate, wie sie z.B. in der US-PS 3 001 973, in den DE-PSen 1 022789, 1 222 067 und 1 027 394 sowie in den DE-OSen 1 929 034 und 2 004 048 beschrieben werden; Urethangruppen aufweisende Polyisocyanate, wie sie z.B. in der DE-PS 752 261 oder in der US-PS 3 394 164 beschrieben werden; acylierte Harnstoffgruppen aufweisende Polyisocyanate gemäß der DE-PS 1 230 778; Biuretgruppen aufweisende Polyisocyanate, wie sie z.B. in der DE-PS 1 101 394, in den US-PSen 3 124 605 und 3 201 372, sowie in der GB-PS 889 050 beschrieben werden; durch Telomerisationsreaktionen hergestellte Polyisocyanate, wie sie z.B. in der US-PS 3 654 106 beschrieben werden; Estergruppen aufweisende Polyisocyanate, wie sie z.B. in den GB-PSen 965 474 und 1 072 956, in der US-PS 3 567 763 und in der DE-PS 1 231 688 genannt werden; Umsetzungsprodukte der obengenannten Isocyanate mit Acetalen gemäß der DE-PS 1 072 385 und polymere Fettsäurereste enthaltene Polyisocyanate gemäß der US-PS 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanaten, einzusetzen. Ferner ist es möglich, beliebige Mischungen der vorgenannten Polyisocyanate zu verwenden.

Bevorzugte Polyisocyanate sind im Allgemeinen die Toluylendiisocyanate und die Diphenylmethandiisocyanate.

Ausgangskomponenten für die Herstellung der Polyurethane sind ferner Verbindungen mit mindestens zwei gegenüber Isocyanaten reaktionsfähigen Wasserstoffatomen mit einem Molekulargewicht in der Regel von 400 - 10 000. Hierunter versteht man neben Aminogruppen, Thiolgruppen oder Carboxylgruppen aufweisenden Verbindungen vorzugsweise Polyhydroxylverbindungen, insbesondere zwei bis acht Hydroxylgruppen aufweisende Verbindungen, speziell solche vom Molekulargewicht 800 bis 10 000, vorzugsweise 1 000 bis 6 000, z.B. mindestens zwei, in der Regel 2 - 8, vorzugsweise aber 2 - 4, Hydroxylgruppen aufweisende Polyester, Polyether, Polythioether, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen, Carbonsäuren. Anstelle der freien Polycarbonsäuren können auch die entsprechenden Polycarbonsäureanhydride oder entsprechenden Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuedimethylester und Terephthalsäure-bis-glykolester.

Als mehrwertige Alkohole kommen z.B. Ethylenglykol, Propylenglykol-(1,2) und - (1,3), Butylenglykol-(1,4) und -(2,3), Hexandiol.-(1,6), Octandiol-(1,8), Neopentyglykol, Cyclohexandimethanol(1,4-Bis-hydroxy-methylcyclohexan), 2-Methyl-1,3 - propandiol, Glycerin, Trimethylolpropan, Hexantriol-(1,2,6), Butantriol-(1,2,4), Trimethyläthan, Pentaerythrit, Chinit, Mannit und Sorbit, Methylglykosit, ferner Diethylenglykol, Triethylenglykol, Tetraethylenglykol, Polyethylenglykole, Dipropy-lenglykol, Polypropylenglykole, Dibutylenglykol und Polybutylenglykole in Frage. Die Polyester können anteilig endständige Carboxylgruppen aufweisen. Auch Polyester aus Lactonen, z.B. ε-Caprolacton oder Hydroxycarbonsäuren, z.B. ω-Hydroxycapronsäure, sind einsetzbar.

Als mehrwertige Alkohole kommen infrage Polyether die mindestens zwei, in der Regel zwei bis acht, vorzugsweise zwei bis drei, Hydroxylgruppen aufweisen. Diese sind an sich bekannt und werden z.B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von BF₃ oder durch Anlagerung dieser Epoxide, gegebenenfalls im Gemisch oder nacheinander, an Startkomponenten mit reaktionsfähigen Wasserstoffatomen wie Wasser, Alkohole, Ammoniak oder Amine, z.B. Ethylenglykol, Propylenglykol-(1,3) oder -(1,2), Trimethylolpropan, 4,4'-Dihydroxy diphenylpropan, Anilin, Ethanolamin oder Ethylendiamin hergestellt. Auch Sucrosepolyether, wie sie z.B. in den DE-ASen 1 176 358 und 1 064 938 beschrieben werden, kommen in Frage. Vielfach sind solche Polyether bevorzugt, die überwiegend (bis zu 90 Gew.%, bezogen auf alle vorhandenen OH-Gruppen im Polyether) primäre OH-Gruppen aufweisen. Auch durch Vinylpolymerisate modifizierte Polyether, wie sie z.B. durch Polymerisation von Styrol und Acrylnitril in Gegenwart von Polyethern entstehen (US-PSen 3 383 351, 3 304 273, 3 523 093, 3 110 695, deutsche Patentschrift 1 152 536), sind geeignet, ebenso OH-Gruppen aufweisende Polybutadiene.

Unter den Polythioethern seien insbesondere die Kondensationsprodukte an Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischether, Polythioetherester oder Polythioetheresteramide.

Als Polyacetale kommen z.B. die aus Glykolen, wie Diethylenglykol, Triethylenglykol, 4,4'-Dioxethoxydiphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diethylenglykol, Triethylenglykol oder Tetraethylenglykol mit Diarylcarbonaten, z.B. Diphenylcarbonat, oder Phosgen hergestellt werden können.

Zu den Polyesteramiden und Polyamiden zählen z.B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnenen, vorwiegend linearen Kondensate.

Auch bereits Urethan- oder Harnstoffgruppen enthaltene Polyhydroxylverbindungen sowie gegebenenfalls modifizierte natürliche Polyole, wie Rizinusöl, Kohlenhydrate oder Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

Vertreter dieser Verbindungen sind z.B. in High Polymers, Vol. XVI, "Polyurethans, Chemistry and Technology", verfaßt von Saunders-Frisch, Interscience Publishers, New York, London, Band I, 1962, Seiten 32 - 42 und Seiten 44 - 54 und Band II, 1964, Seiten 5 -6 und 198 - 199, sowie im Kunststoff-Handbuch, Band VII, Vieweg-Höchtlen, Carl-Hanser-Verlag, München, 1966, z.B. auf den Seiten 45 - 71, beschrieben.

Selbstverständlich können Mischungen der obengenannten Verbindungen mit mindestens zwei gegenüber Isocyanaten reaktionsfähigen Wasserstoffatomen mit einem Molekulargewicht von 400 - 10 000, z.B. Mischungen von Polyethern, eingesetzt werden.

Als gegebenenfalls einzusetzende Ausgangskomponenten kommen auch Verbindungen mit mindestens zwei gegenüber Isocyanaten reaktionsfähigen Wasserstoffatomen von einem Molekulargewicht 32 - 400 in Frage. Auch in diesem Fall versteht man hierunter Hydroxylgruppen und/oder Aminogruppen und/oder Thiolgruppen und/oder Carboxylgruppen aufweisende Verbindungen, vorzugsweise Verbindungen, die als Kettenverlängerungsmittel oder Vernetzungsmittel dienen. Diese Verbindungen weisen in der Regel 2 bis 8 gegenüber Isocyanaten reaktionsfähige Wasserstoffatome auf, vorzugsweise 2 oder 3 reaktionsfähige Wasserstoffatome.

Als Beispiele für derartige Verbindungen seien genannt:
Ethylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Glyzerin, Trimethylolpropan, Hexantriol-(1,2,6), Trimethyloläthan, Pentaerythrit, Chinit, Mannit und Sorbit, Diethylenglykol, Triethylenglykol, Tetraethylenglykol, Polyethylenglykole mit einem Molekulargewicht bis 400, Dipropylenglykol, Polypropylenglykole mit einem Molekulargewicht bis 400, Dibutyllenglykol, Polybutylenglykole mit einem Molekulargewicht bis 400, 4,4'-Dihydroxy-diphenylpropan, Di-hydroxymethyl-hydrochinon, Äthanolamin, Diäthanolamin, Triäthanolamin, 3-Aminopropanol, Ethylendiamin, 1,3-Diaminopropan, 1-Mercapto-3-aminopropan, 4-Hydroxy- oder -Amino-phthalsäure, Bernsteinsäure, Adipinsäure, Hydrazin, N,N'-Dimethylhydrazin, 4,4'-Diaminodiphenylmethan, Toluylendiamin, Methylen-bis-chloranilin, Methylen-bisanthranilsäueester, Diaminobenzoesäureester und die isomeren Chlorphenylendiamine.

Auch in diesem Fall können Mischungen von verschiedenen Verbindungen mit mindestens zwei gegenüber Isocyanaten reaktionsfähigen Wasserstoffatomen mit einem Molekulargewicht von 32 - 400 verwendet werden.

Es können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) direkt in situ in den oben genannten, Hydroxylgruppen aufweisenden Verbindungen ablaufen läßt. Derartige Verfahren sind beispielsweise in den DE-ASen 1 168 075 und 1 260 142, sowie den DE-OSen 2 324 134, 2 423 984, 2 512 385, 2 513 815, 2 550 797, 2 550 833 und 2 550 862 beschrieben. Es ist aber auch möglich, gemäß US-PS 3 869 413 bzw. DE-OS 2 550 860 eine fertige wässrige Polymerdispersion mit einer Polyhydroxylverbindung zu vermischen und anschließend aus dem Gemisch das Wasser zu entfernen.

Bei der Auswahl der für die Herstellung des Polyurethans verwendeten höhermolekularen Polyolkomponente ist zu beachten, daß das fertige Polyurethan in Wasser nicht quellbar sein soll. Die Verwendung eines Überschusses an Polyhydroxylverbindungen mit Ethylenoxideinheiten (Polyethylenglykolpolyether oder Polyester mit Diethylen- oder Triethylenglykol als Diolkomponente) ist daher zu vermeiden.

Besonders hervorgehoben zur Herstellung der Formkörper seien thermoplastische Elastomere. Dies sind Werkstoffe, die elastomere Phasen in thermoplastisch verarbeitbaren Polymeren entweder physikalisch eingemischt oder chemisch eingebunden enthalten. Man unterscheidet Polymerblends, in denen die elastomeren Phasen Bestandteil des polymeren Gerüsts sind. Durch den Aufbau der thermoplastischen Elastomere liegen harte und weiche Bereiche nebeneinander vor. Die harten Bereiche bilden dabei eine kristalline Netzstruktur oder eine kontinuierliche Phase deren Zwischenräume von elastomeren Segmenten ausgefüllt sind. Aufgrund dieses Aufbaus haben diese Werkstoffe kautschukähnliche Eigenschaften.

Man kann 5 Hauptgruppen der thermoplastischen Elastomere unterscheiden:
1. Copolyester
2. Polyether-Block-Amide (PEBA)
3. Thermoplastische Polyurethane (TPU)
4. Thermoplastische Polyolefine (TPO)
5. Styrol-Block Copolymere

Geeignete Copolyester (segmentierte Polyesterelastomere) sind beispielsweise aus einer Vielzahl wiederkehrender, kurzkettiger Estereinheiten und langkettiger Estereinheiten, die durch Esterbindungen vereinigt sind, aufgebaut, wobei die kurzkettigen Estereinheiten etwa 15-65 Gew.% des Copolyesters ausmachen und die Formel I haben. 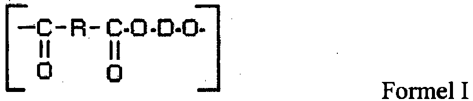 in welcher
- R: für einen zweiwertigen Rest einer Dicarbonsäure steht, der ein Molekulargewicht von unter etwa 350 hat,
- D: für einen zweiwertigen Rest eines organischen Diols steht, der ein Molekulargewicht von unter etwa 250 hat,

Die langkettigen Estereinheiten machen etwa 35-85 Gew.-% des Copolyesters aus und haben die Formel II 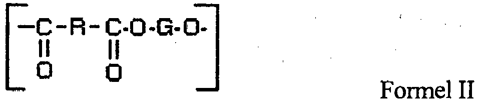 in welcher
- R: für einen zweiwertigen Rest einer Dicarbonsäure steht, der ein Molekulargewicht von unter etwa 350 hat,
- G: für einen zweiwertigen Rest eines langkettigen Glyköls steht, das ein durchschnittliches Molekulargewicht von etwa 350 bis 6000 hat.

Die erfindungsgemäß verwendbaren Copolyester sind herstellbar, indem man miteinander a) eine oder mehrere Dicarbonsäuren, b) ein oder mehrere lineare, langkettige Glykole und c) ein oder mehrere niedermolekulare Diole polymerisiert.

Die Dicarbonsäuren für die Herstellung des Copolyesters können aromatisch, aliphatisch oder cycloaliphatisch sein. Die bevorzugten Dicarbonsäuren sind die aromatischen Säuren mit 8-16 C-Atomen, insbesondere Phenylendicarbonsäuren, wie Phthal-, Terephthal- und Isophthalsäure.

Die niedermolekularen Diole für die Umsetzung zur Bildung der kurzkettigen Estereinheiten der Copolyester gehören den Klassen der acyclischen, alicyclischen und aromatischen Dihydroxyverbindungen an. Die bevorzugten Diole haben 2-15 C-Atome, wie Ethylen-, Propylen-, Tetramethylen-, Isobutylen-, Pentamethylen-, 2,2-Di-methyltrimethylen-, Hexamethylen- und Decamethylenglykole, Dihydroxycyclohexan, Cyclohexandimethanol, Resorcin, Hydrochinon und dergleichen. Zu den Bisphenolen für den vorliegenden Zweck gehören Bis-(p-hydroxy)-diphenyl, Bis-(p-hydroxyphenyl)-methan, Bis-(p-hydroxyphenyl)-ethan und Bis-(p-hydroxyphenyl)-propan.

Die langkettigen Glycole zur Herstellung der weichen Segmente der Copolyester haben vorzugsweise Molekulargewichte von etwa 600 bis 3000. Zu ihnen gehören Poly-(alkylenether)-glycole, bei denen die Alkylengruppen 2-9 Kohlenstoffatome aufweisen.

Auch Glycolester von Poly-(alkylenoxid)-dicarbonsäuren können als langkettiges Glycol Verwendung finden.

Auch Polyesterglycole können als langkettiges Glycol Verwendung finden.

Zu den langkettigen Glycolen gehören auch Polyformale, die durch Umsetzung von Formaldehyd mit Glycolen erhalten werden. Auch Polythioetherglycole sind geeignet. Polybutadien- und Polyisoprenglycole, Mischpolymere derselben und gesättigte Hydrierungsprodukte dieser Materialien stellen zufriedenstellende langkettige polymere Glycole dar.

Verfahren zur Synthese derartiger Copolyester sind aus DOS 2 239 271, DOS 2 213 128, DOS 2 449 343 und US-P 3 023 192 bekannt.

Geeignete Copolyester sind z.B. unter den Handelsnamen ^{®}Hytrel der Fa. Du Pont, ^{®}Pelpren der Fa. Toyobo^{®}, Arnitel der Fa. Akzo,^{®}Ectel der Fa. Eastman Kodak und ^{®}Riteflex der Fa. Hoechst erhältlich.

Geeignete Polyether-Blockamide sind beispielsweise solche, die aus Polymerketten bestehen, die aus wiederkehrenden Einheiten entsprechend der Formel I aufgebaut sind. 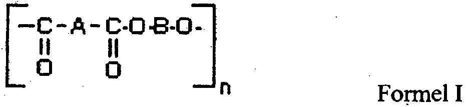 in welcher
- A: die von einem Polyamid mit 2 Carboxylendgruppen durch Verlust der letzteren abgeleitete Polyamidkette ist und
- B: die von einem Polyoxyalkylenglycol mit endständigen OH-Gruppen durch Verlust der letzteren abgeleitete Polyoxyalkylenglycolkette ist und
- n: die Zahl der die Polymerkette bildenden Einheiten ist.

Als Endgruppen stehen dabei bevorzugt OH-Gruppen oder Reste von Verbindungen die die Polymerisation abbrechen.

Die Dicarbonsäurepolyamide mit den endständigen Carboxylgruppen werden auf bekannte Weise erhalten, so z.B. durch Polykondensation eines oder mehrerer Lactame oder/und einer oder mehrerer Aminosäure, ferner durch Polykondensation einer Dicarbonsäure mit einem Diamin, jeweils in Gegenwart eines Überschusses einer organischen Dicarbonsäure, vorzugsweise mit endständigen Carboxylgruppen. Diese Carbonsäuren werden während der Polykondensation Bestandteil der Polyamidkette und lagern sich insbesondere an den Ende derselben an, wodurch man ein α-ω-dicarbonsaures Polyamid erhält. Ferner wirkt die Dicarbonsäure als Kettenabbruchmittel, weshalb sie auch im Überschuß eingesetzt wird.

Das Polyamid kann erhalten werden, ausgehend von Lactamen und/oder Aminosäuren mit einer Kohlenwasserstoffkette bestehend aus 4-14 C-Atomen, wie z.B. von Caprolactam, Oenantholactam, Dodekalactam, Undekanolactam, Dekanolactam, 11-Amino-undekano oder 12-Aminododekansäure.

Als Beispiele für Polyamide, wie sie durch Polykondensation einer Dicarbonsäure mit einem Diamin entstehen, können genannt werden die Kondensationsprodukte aus Hexamethylendiamin mit Adipin-, Azelain-, Sebacin-, und 1,12-Dodecandisäure, sowie die Kondensationsprodukte aus Nonamethylendiamin und Adipinsäure.

Bei den zur Synthese des Polyamids, das heißt, einerseits zur Fixierung jeweils einer Carboxylgruppe an jedem Ende der Polyamidkette und andererseits als Kettenabbruchmittel verwendeten Dicarbonsäure kommen solche mit 4-20 C-Atomen in Frage, insbesondere Alkandisäuren, wie Bernstein-, Adipin-, Kork-, Azelain-, Sebacin-, Undekandi- oder Dodekandisäure, ferner cycloaliphatische oder aromatische Dicarbonsäure, wie Terephthal- oder Isphthal- oder Cyclohexan-1,4-dicarbonsäure.

Die endständigen OH-Gruppen aufweisenden Polyoxyalkylenglycole sind unverzweigt oder verzweigt und weisen einen Alkylenrest mit mindestens 2 C-Atomen auf. Insbesondere sind dies Polyoxyethylen-, Polyoxypropylen- und Polyoxytetramethylenglycol, sowie Copolymerisate davon.

Das Durchschriittsmolekulargewicht dieser OH-Gruppen-terminierten Polyoxyalkylenglycole kann sich in einem großen Bereich bewegen, vorteilhaft liegt es zwischen 100 und 6000, insbesondere zwischen 200 und 3000.

Der Gewichtsanteil des Polyoxyalkylenglycols, bezogen auf das Gesamtgewicht des zur Herstellung des PEBA-Polymeren verwendeten Polyoxyalkylenglycols und Dicarbonsäurepolyamids beträgt 5-85 % vorzugsweise 10-50 %.

Verfahren zur Synthese derartiger PEBA-Polymerer sind aus FR-PS 7 418 913 (Nr. der Veröffentlichung 2 273 021), DOS 2 802 989, DOS 2 837 687, DOS 2 523 991, EP-S 0 095 893, DOS 2 712 987 beziehungsweise DOS 2 716 004 bekannt.

Bevorzugt geeignet sind solche PEBA-Polymere, die im Gegensatz zu den vorher beschriebenen, statistisch aufgebaut sind. Zur Herstellung dieser Polymere wird ein Gemisch aus
1. einer oder mehrerer polyamidbildender Verbindungen aus der Gruppe der ω-Aminocarbonsäuren beziehungsweise Lactame mit mindestens 10 Kohlenstoffatomen,
2. einem ∝-ω-Dihydroxy-Polyoxyalkylenglycol,
3. mindestens einer organischen Dicarbonsäure
   in einem Gewichtsverhältnis von 1:(2+3) zwischen 30:70 und 98:2, wobei in (2+3) Hydroxyl- und Carbonylgruppen in äquivalenten Mengen vorliegen, in Gegenwart von 2 bis 30 Gewichtsprozent Wasser, bezogen auf die polyamidbildenden Verbindungen der Gruppe 1, unter dem sich einstellenden Eigendruck auf Temperaturen zwischen 23°C und 30°C erhitzt und anschließend nach Entfernen des Wassers unter Ausschluß von Sauerstoff bei Normaldruck oder unter vermindertem Druck bei 250 bis 280°C weiterbehandelt werden.

Solche bevorzugt geeignete PEBA-Polymere sind z.B. in DE-OS 2 712 987 beschrieben.

Geeignete und bevorzugt geeignete PEBA-Polymere sind z.B. unter den Handelsnamen ^{®}PEBAX der Fa. Atochem, ^{®}Vestamid der Fa. Hüls AG, ^{®}Grilamid der Fa. EMS-Chemie und ^{®}Kellaflex der Fa. DSM erhältlich.

Die Formkörper enthalten Wirkstoff in Konzentrationen von 1 bis 20 Gew.-%, bevorzugt von 5 bis 20 Gew.%, besonders bevorzugt um 10 Gew.-%.

Im Fall von Halsbändern liegt die Konzentration der Wirkstoffe bevorzugt bei 1 bis 15 %; im Fall von Medaillons, Anhängern und Ohrmarken bevorzugt bei 5 bis 20 %, im Fall von Folien, Klebestreifen bevorzugt bei 0,1 bis 5 %.

Imidacloprid liegt in Mischung mit den synthetischen Pyrethroiden Flumethrin (3-[2-(4-Chlorphenyl)-2-chlorvinyl]-2,2-dimethyl-cyclo-propancarbonsäure [(∝-cyano-4-fluor-3-phenoxy)-benzyl]-ester) oder Permethrin (3-Phenoxybenzyl(±)-cis, trans-3-(2,2-dichlorvinyl)-2,2-dimethylcyclopropancarboxylat) vor.

Die Formkörper können weiterhin die für Kunststoffe üblichen Additive enthalten. Übliche Additive sind beispielsweise Pigmente, Stabilisatoren, Fließmittel, Gleitmittel, Entformungsmittel.

In den folgenden Beispielen wird Imidacloprid eingesetzt. Es handelt sich nicht um erfindungsgemäße Beispiele, da darin keine Mischung von Imidacloprid mit Flumethrin oder Permethrin verwendet wird. Gleichwohl geben sie einen technologischen Hintergrund zur Anwendung von Imidacloprid.

### Beispiel 1

### SC-(Suspensionskonzentrat) Formulierung:

| | |
|---|---|
| 368 g | Imidacloprid |
| 35 g | Blockpolymer aus Emulgator |
| | Ethylenoxid- und Propylenoxid |
| 12 g | Ditolylethersulfonat-Formaldehyd-Kondensat (Emulgator) |
| 3,5 g | wasserlöslicher Polyvinylalkohol |
| 58,0 g | NH₄Cl |
| 116,0 g | Harnstoff |
| 1,2 g | (37 %ige wäßrige Salzsäure) |
| 4,6 g | Xanthan-gum |
| 560,5 g | destilliertes Wasser |

### Beispiel 2

### WP (dispergierbares Pulver)-Formulierung:

| | |
|---|---|
| 25,0 g | Imidacloprid |
| 1,0 g | Diisobutyl-naphthalin-Na-sulfonat |
| 10,0 g | n-Dodecylbenzylsulfonsäure Calcium |
| 12,0 g | hochdisperser Kieselsäure-haltiger Alkylarylpolyglykolether |
| 3,0 g | Ditolylethersulfonat-Formaldehyd-Kondensat (Emulgator) |
| 2,0 g | ^{®}Baysilon-E, ein siliconhaltiger Entschäumer der Fa. Bayer AG |
| 2,0 g | feindisperses Siliciumdioxid und |
| 45,0 g | Kaolin |

### Beispiel 3

### SL-(wasserlösliche Konzentrat)-Formulierung

| | |
|---|---|
| 18,3 g | Imidacloprid |
| 2,5 g | neutraler Emulgator auf Basis Alkylarylpolyglykolether |
| 3,5 g | Natriumsulfobernsteinsäurediisooctylester |
| 38,4 g | Dimethylsulfoxid und |
| 37,5 g | 2-Propanol |

### Beispiel 4

### SL-(wasserlösliche Konzentrat)-Formulierung

| | |
|---|---|
| 185, g | Imidacloprid |
| 5,0 g | Natriumsulfonbernsteinsäurediisooctylester und |
| 76,5 g | Dimethylsulfoxid |

werden einer 100 g Schampoo-Formulierung bestehend aus

| | |
|---|---|
| 44,4 Gew.-% | Marlon AT 50, ein Triethanolaminsalz von Alkylbenzolsulfonsäuren der Fa. Hüls AG |
| 11,1 Gew.-% | Marlon A 350, Natriumsalz von Alkylbenzolsulfonsäuren der Fa. Hüls AG |
| 3,0 Gew.-% | Kondensationsprodukt von Ölsäuren und Diethanolamin der Fa. Hüls AG und |
| 41,5 Gew.-% | Polyethylenglykol |

beigemengt.

### Beispiel 5

### Spray-Formulierung bestehend aus

| | |
|---|---|
| 2,0 g | Imidacloprid |
| 10,0 g | Dimethylsulfoxid |
| 35,0 g | 2-Propanol und |
| 53,0 g | Aceton |

### Beispiel 6

### pour-on-Formulierung

| | |
|---|---|
| 20,3 g | Imidacloprid |
| 1,8 g | Polyvinylalkohol |
| 1,8 g | Blockcopolymerisat auf Basis Ethylenoxid und Propylenoxid |
| 0,26 g | Xanthan Gum |
| 9,0 g | Glycerin |
| 59,2 | destilliertes Wasser |

### Beispiel 7

| | | |
|---|---|---|
| Zusammensetzung: | Imidacloprid | 10,00 g |
| | Di-n-butyladipat | 21,10 g |
| | Diethylhexylphthalat | 9,10 g |
| | Epoxidiertes Sojabohnenöl | 2,30 g |
| | Stearinsäure | 0,80 g |
| | PVC | 56,70 g |

Herstellung: Die homogene Mischung aus Imidacloprid und PVC wird in einem Mischer mit dem Gemisch aus Di-n-butyladipat, Diethylhexylphthalat und Epoxidiertem Sojabohnenöl benetzt. Man mischt solange, bis die Mischung homogen ist. Dabei fördert Erwärmen, z.B. infolge einer Erhöhung der Tourenzahl des Mischers, das Einziehen der Weichmachermischung in das PVC. Nach der anschließenden homogenen Verteilung der Stearinsäure wird die Mischung im Spritzguß zu Hundehalsbändern geformt.

### Beispiel 8

| | | |
|---|---|---|
| Zusammensetzung: | Imidachloprid | 10,00 g |
| | Epoxidiertes Sojabohnenöl | 2,30 g |
| | Stearinsäure | 0,80 g |
| | Acetyltributylcitrat | 30,20 g |
| | PVC | 56,70 g |

Herstellung: Das Gemisch aus Acetyltributylcitrat und Epoxidiertem Sojabohnenöl wird in einem Mischer auf die homogene Mischung aus Imidacloprid und PVC aufgebracht. Erwärmen fördert beim Mischen das Einziehen des Weichmachergemisches in das PVC; man mischt solange, bis die Mischung homogen ist. Die Mischung wird in herkömmlicher Weise zu Hundehalsbändern extrudiert.

### Beispiel 9

| | | |
|---|---|---|
| Zusammensetzung: | Imidachloprid | 20,00 g |
| | Epoxidiertes Sojabohnenöl | 2,30 g |
| | Stearinsäure | 0,80 g |
| | Acetyltributylcitrat | 30,20 g |
| | PVC | 56,70 g |

### Herstellung: wie Beispiel 8

### Beispiel 10

| | | |
|---|---|---|
| Zusammensetzung: | Imidachloprid | 7,50 g |
| | Epoxidiertes Sojabohnenöl | 10;00 g |
| | Stearinsäure | 0,80 g |
| | Acetyltributylcitrat | 15,00 g |
| | PVC | 66,70 g |

### Herstellung: wie Beispiel 8

### Beispiel 11

| | | |
|---|---|---|
| Zusammensetzung: | Imidachloprid | 10,00 g |
| | Epoxidiertes Sojabohnenöl | 2,30 g |
| | Stearinsäure | 0,80 g |
| | Triacetin | 15,00 g |
| | PVC | 71,90 g |

Herstellung: Das Gemisch aus Triacetin und Epoxidiertem Sojabohnenöl wird in einem Mischer auf die homogene Mischung aus PVC und Imidacloprid aufgebracht. Dabei fördert Erwärmen, z.B. infolge einer Erhöhung der Tourenzahl des Mischers, das Einziehen des Weichmachers in das PVC. Nach homogener Untermischung der Stearinsäure wird die Mischung auf einem Extruder zu Platten extrudiert, aus denen Medaillons (=Anhänger für Halsbänder) ausgestanzt werden.

### Beispiel 12

| | | |
|---|---|---|
| Zusammensatzung: | Imidacloprid | 5,00 g |
| | Polyetherblockamid (Pebax^{®}) | 94,50 g |

Herstellung: Der Wirkstoff wird in einem Intensivmischer auf den Träger aufgebracht und die Mischung in Spritzguß zu Hurtdehalsbändern geformt.

### Beispiel 13

| | | |
|---|---|---|
| Zusammensetzung: | Imidacloprid | 10,00 g |
| | Mittelkettige Triglyceride | 15,00 g |
| | hochdisperses Siliciumdioxid | 0,50 g |
| | Polyetherblockamid (Pebax^{®}) | 74,50 g |

Herstellung: Mittelkettige Triglyceride werden in einem Mischer auf die homogene Mischung aus Imidacloprid und Polyetherblockamid aufgebracht. Erwärmen fördert dabei das Einziehen der mittelkettigen Triglyceride in das Polyetherblockamid. Zur Verbesserung der Fließfähigkeit wird vor der Extrusion der Mischung hochdisperses Siliciumdioxid homogen untergemischt. Es werden Platten extrudiert, aus denen Medaillons (=Anhänger für Halsbänder) ausgestanzt werden.

### Beispiel 14

| | | |
|---|---|---|
| Zusammensetzung: | Imidacloprid | 10,00 g |
| | Styrol-Butylen-Block-Copolymer (Thermoplast^{®}K) | 90,00 g |

Herstellung: Der Wirkstoff wird in einem Intensivmischer auf den Träger aufgebracht und die Mischung im Spritzguß zu Halsbändern geformt.

### Beispiel 15

| | | |
|---|---|---|
| Zusammensetzung: | Imidacloprid | 5,00 g |
| | Copolyester (Hytrel^{®}) | 95,00 g |

Herstellung: Die Mischung wird in herkömmlicher Weise zu Hundehalsbändern extrudiert.

### Beispiel 16

| | | |
|---|---|---|
| Zusammensetzung: | Imidacloprid | 10,00 g |
| | Polyetherblockamid (Pebax^{®}) | 90,00 g |

Herstellung: Die homogene Mischung wird auf einem Extruder zu Platten extrudiert, aus denen Medaillons (= Anhänger für Halsbänder) ausgestanzt werden.

### Beispiel 17

| | | |
|---|---|---|
| Zusammensetzung: | Imidacloprid | 10,00 g |
| | mittelkettige Triglyceride | 30,00 g |
| | hochdisperses Siliciumdioxid | 0,50 g |
| | Polyetherblockamid (Pebax^{®}) | 59,50 g |

### Herstellung: wie Beispiel 13

### Beispiel A

1 ml der im Beispiel 1 angegebenen SC-Formulierung wurde als Lösung pour-on auf die Schulter eines mit 200 Flöhen infestierten Hundes aufgetragen. Das Versuchstier konnte sofort von adulten Flöhen befreit werden. Die erfindungsgemäße Behandlung führt zu einer 100 %igen Mortalitätsrate der Flöhe.

### Anwendungsbeispiel A

1 ml der in Beispiel 1 beschriebenen Formulierung wurde in 11 Wasser verdünnt und mit dieser Lösung Hunde die mit Flöhen infestiert waren tropfnaß begossen. Es wurden folgende Ergebnisse erhalten:

| Zeitraum Tag | Anzahl der Flöhe pro Hund | | % Wirkung |
|---|---|---|---|
| | unbehandelt | behandelt | |
| -1 Infestation mit 100 Flöhen | | | |
| 0 Behandlung und Zählung | 30 | 0 | 100 |
| 5, 8 Infestation mit 100 Flöhen | | | |
| 9 Zählung | 56 | 0 | 100 |
| 15 Infestation mit 100 Flöhen | | | |
| 16 Zählung | 76 | 0 | 100 |
| 19 Infestation mit 100 Flöhen (unbehandeltes Tiere) | | | |
| 250 Flöhen (behandelte Tiere | | | |
| 20 Zählung | 39 | 0 | 100 |
| 26 Infestation mit 100 Flöhen | | | |
| 27 Zählung | 43 | 0 | 100 |

### Anwendungsbeispiel B

1 ml der Lösung gemäß Beispiel 1 wurden auf die Schulter eines Hundes gegeben. Das Tier wurde nach 2 und nach 6 Tagen der Behandlung mit 200 Flöhen infestiert. Jeweils am Tag 3 und am Tag 7 nach Behandlung wurden die am Hund verbliebenen Flöhe gezählt. Es konnten keine lebenden Flöhe gefunden werden. Die Wirkung war 100%.

## Patentansprüche

1. Verwendung von Imidacloprid (= 1-[(6-Chlor-3-pyridinyl)methyl]-N-nitro-2-imidazolidinimin) zur Herstellung von Mitteln zur nicht-systemischen Bekämpfung von parasitierenden Insekten aus der Gruppe der Flöhe, Läuse und Fliegen an Menschen und Tieren in Mischung mit den synthetischen Pyrethroiden Flumethrin oder Permethrin.

## Claims

1. Use of imidacloprid (=1-[(6-chloro-3-pyridinyl)méthyl]-N-nitro-2-imidazolidinimine) for preparing compositions for nonsystemic control of parasitic insects from the group of fleas, lice and flies on humans and animals in mixture with the synthetic pyrethroids flumethrin or permethrin.

## Revendications

1. Utilisation d'imidaclopride (=1-[(6-chloro-3-pyridinyl)méthyl]-N-nitro-2-imidazolidinimine) pour la préparation de compositions pour la lutte non systémique contre les insectes parasites du groupe des puces, des poux et des mouches sur l'homme et les animaux en mélange avec des pyréthroïdes synthétiques fluméthrine ou perméthrine.
